# FASCICULE DE BREVET EUROPEEN

(11) **EP 3 423 408 B1**
(45) Date de publication et mention de la délivrance du brevet: **27.04.2022**
(21) Numéro de dépôt: 17711714.0
(22) Date de dépôt: 01.03.2017
(51) Int. Cl.: C01F 17/00, A61K 6/00

(54) **SUSPENSION COLLOIDALE DE NANOPARTICULES A BASE DE FLUORURE DE TERRE RARE**
KOLLOIDALE SUSPENSION VON NANOPARTIKELN AUF SELTENERDFLUORIDBASIS
COLLOIDAL SUSPENSION OF RARE EARTH FLUORIDE-BASED NANOPARTICLES

(30) Priorité: 01.03.2016 FR 1651699
(43) Date de publication de la demande: 09.01.2019
(73) Titulaire: MATHYM, 69410 Champagne-Au-Mont-D'or (FR)
(72) Inventeur: FAURE, Anne-Charlotte, 69100 Villeurbanne (FR); ALBERICI, Julien, 69410 Champagne AU Mont D'Or (FR)
(74) Mandataire: Schmidt, Martin Peter
(86) Numéro de dépôt international: PCT/FR2017/050455
(87) Numéro de publication internationale: WO 2017/149242

(56) Documents cités:
- EP-A1- 1 502 570
- US-A1- 2008 092 994
- FRÉDÉRIC CHAPUT ET AL: "Rare Earth Fluoride Nanoparticles Obtained Using Charge Transfer Complexes: A Versatile and Efficient Route toward Colloidal Suspensions and Monolithic Transparent Xerogels", LANGMUIR, vol. 27, no. 9, 3 mai 2011 (2011-05-03), pages 5555-5561, XP055302755, US ISSN: 0743-7463, DOI: 10.1021/la105070p

## Description

### Domaine technique de l'invention

L'invention concerne le domaine de la chimie minérale, à savoir le domaine des fluorures de terre rare. Elle concerne plus particulièrement, des suspensions colloïdales de fluorure de terre rare sous forme de nanoparticules. Plus particulièrement, elle concerne un procédé de synthèse de nanoparticules de fluorure de terre rare, la fonctionnalisation de ces nanoparticules et leur utilisation comme nanocharges dans des composites dentaires polymérisables, dans des matériaux de restauration dentaires polymérisables tels que les matériaux de comblement dentaire et les adhésifs dentaires.

### Etat de la technique

Les fluorures de terre rare présentent des propriétés optiques, magnétiques, chimiques et structurales particulières. Certaines applications en tirent profit, notamment comme radio-opacifiant biomédical. Dans ces applications, les fluorures de terre rare sont souvent utilisés sous forme de poudre sèche.

Les procédés de fabrication de nanoparticules de fluorure de terre rare mis en oeuvre à l'échelle industrielle conduisent à des nanoparticules polydisperses, de faible cristallinité et relativement impures. Ils conduisent uniquement à des nanoparticules sous forme de poudre, présentant des morphologies et propriétés assez diverses.

D'une manière générale, les fluorures de terre rare sont insolubles dans l'eau ; pour toute utilisation intracorporelle, il faut veiller à ne pas les contaminer avec des traces de solvant toxique.

Lezhnina et al. (Adv. Func. Mat. 2006, vol. 16, n°7, pp. 935-942) ont décrit un procédé d'obtention de fluorure de terre rare mettant en oeuvre une réaction sous pression à chaud (180°C en autoclave) entre Ln(NO₃)₃ et NH₄F; Ln³⁺ ou « Ln » symbolisant un lanthanide. Par cette méthode, seuls les fluorures des terres rares comprises entre le lanthane et le gadolinium peuvent être préparés tels que des particules de LaF₃ de 15-30 nm, de GdF₃ de 30-70 nm ou encore de EuF₃ de 25-40 nm. Toutefois, pour des terres rares comprises entre le gadolinium et le lutétium, cette méthode conduit uniquement à l'obtention de fluorures de terre rare mixtes NH₄LnF₄. Par ailleurs, de par les matières premières utilisées, ce procédé ne permet pas d'obtenir des solutions colloïdales à fort extrait sec après synthèse et purification.

FR 2 946 973 tout comme la publication de F. Chaput et al., « Rare earth fluoride nanoparticles obtained using charge transfer complexes: a versatile and efficient route toward colloidal suspensions and monolithic transparent xerogels », Langmuir, vol. 27 n°9 (2011) p.5555-5561, divulgue un procédé de préparation de nanoparticules à base de fluorure de terre rare par réaction d'une solution d'un sel de terre rare dans un solvant protique tel que le méthanol, l'isopropanol et l'éthanol avec une solution d'un complexe de transfert de charge de formule (I), 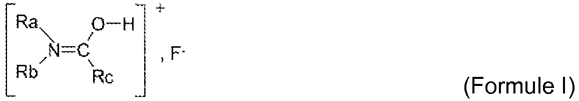 cette réaction étant suivie du chauffage du mélange réactionnel. Ce document enseigne que la température et le temps de chauffage du mélange réactionnel influent sur la cristallinité ; la taille des particules obtenues augmente avec le temps de chauffage du mélange.

Cependant, les suspensions colloïdales obtenues après purification et fonctionnalisation ne sont pas stables à forte concentration, les particules s'agrégeant au-delà d'une concentration massique supérieure à 20%. De plus, les particules décrites dans FR 2 946 973 sont mal cristallisées, et leur fonctionnalisation ultérieure est compliquée. A cause de leur instabilité, ces suspensions de nanoparticules sont utilisées dans une concentration inférieure à 20% massique, de manière à éviter toute agrégation et ainsi à conserver l'intégrité de la suspension.

Le problème que la présente invention cherche à résoudre est de proposer des suspensions colloïdales de nanoparticules de fluorure de terre rare, individualisées (i.e. non agglomérées), facilement fonctionnalisables et stables, dans lesquelles ces nanoparticules sont présentes à une concentration supérieure à 20% massique, ont une taille donnée en D50 inférieure à 300 nm, de préférence inférieure à 200 nm et dont la composition chimique, la cristallinité et la pureté sont maitrisées.

La présente invention a aussi pour objet de fournir un procédé de fabrication de suspensions colloïdales de nanoparticules de fluorure de terre rare qui soit simple, sûr, rapide, facile à mettre en oeuvre, peu coûteux et pouvant s'affranchir de l'emploi de solvant toxique.

Un autre objectif de l'invention est de proposer des suspensions colloïdales de nanoparticules de fluorure de terre rare prêtes à l'emploi et facilement fonctionnalisables.

Un autre objectif de l'invention est de proposer des suspensions colloïdales de nanoparticules de fluorure de terre rare fonctionnalisées, pouvant notamment être utilisées comme nanocharges dans des composites dentaires polymérisables ou dans des matériaux de restauration dentaires polymérisables, tels que les matériaux de comblement dentaire et les adhésifs dentaires.

### Objets de l'invention

La présente invention concerne une suspension colloïdale de nanoparticules de fluorure de terre rare dans laquelle lesdites nanoparticules ont une taille donnée en D50 inférieure à 300 nm, de préférence inférieure à 200 nm, et encore plus préférentiellement inférieure à 100 nm, et sont présentes à une concentration supérieure à 20%, de préférence supérieure à 23%, et encore plus préférentiellement supérieure à 25%, le pourcentage étant basé sur le poids de la suspension.

Avantageusement, la suspension colloïdale de nanoparticules de fluorure de terre rare est une suspension de nanoparticules de fluorure de terre rare fonctionnalisées.

Dans un mode de réalisation, le solvant dispersant les nanoparticules de fluorure de terre rare fonctionnalisées ou non fonctionnalisées de la suspension colloïdale est choisi parmi les hydrocarbures, les hydrocarbures halogénés, l'eau, les alcools, les éthers, les glycols, les éthers de glycol, les aldéhydes, les cétones, les acides carboxyliques, les esters, les esters de carbonate, les aminés, les amides, les thiols, les solvants organosulfurés, les monomères acryliques et méthacryliques, les monomères vinyliques, les monomères époxydes, les huiles silicone, les organosilanes, les polycarbosilanes et les alkoxysilanes.

Avantageusement, le solvant dispersant les nanoparticules de fluorure de terre rare fonctionnalisées de la suspension colloïdale est choisi parmi l'acétone, l'eau, l'éthanol, le bisphénol A glycerolate diméthacrylate (Bis-GMA), le bisphénol A glycol éthoxylé diméthacrylate (Bis-EMA), le triéthylène glycol diméthacrylate (TEGDMA), le 2-hydroxyéthyl méthacrylate (HEMA), le 1,6-hexanediol diméthacrylate (HDDMA), le 1,10-décanediol diméthacrylate (D3MA) ou un mélange de ces solvants.

Si la suspension colloïdale de nanoparticules de fluorure de terre rare est destinée à être utilisée pour les applications médicales, et en particulier intracorporelles comme en médecine dentaire, on préfère que le(s) solvant(s) utilisé(s) pour la réaction entre le sel de terre rare et le complexe de transfert de charge soi(en)t non toxique, et que la phase liquide dispersant les nanoparticules soit choisie parmi les monomères ou oligomères utilisés éventuellement dans la polymérisation (si ladite suspension colloïdale est destinée à être polymérisée) et contienne ( dans tous les cas) essentiellement ou exclusivement des solvants non toxiques compatibles avec leur application, tels que l'eau et l'éthanol.

Par « solvant non toxique » on entend tout solvant présentant peu ou pas de risques pour la santé, tel que l'eau ou l'éthanol.

Avantageusement, la suspension colloïdale de nanoparticules de fluorure de terre rare peut être utilisée dans la fabrication d'un matériau de restauration dentaire, et notamment pour le comblement dentaire.

Un autre objet de l'invention est un procédé de préparation d'une suspension colloïdale de nanoparticules de fluorure de terre rare dans lequel :
a. on prépare une solution, de préférence aqueuse, d'un sel de la terre rare correspondante,
b. on prépare une solution d'un complexe de transfert de charge de formule (I) 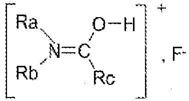 par réaction d'un amide de formule II 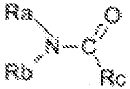 avec une source d'ions fluorure, de préférence de l'acide fluorhydrique, du fluorure de tétrabutylammonium, du trihydrofluorure de triéthylamine ou de l'acide fluorosilicique,
   où : Ra et Rb, identiques ou différents, représentent, chacun indépendamment l'un de l'autre, un groupe (C1-C6)alkyle, (C3-C7)cycloalkyle, phényle ou hétérocycloalkyle de 5 ou 6 chainons, ledit groupe pouvant éventuellement être substitué, ou bien Ra et Rb sont liés entre eux et forment une chaine alkylène, comprenant de 2 à 6 atomes de carbone, éventuellement substituée, et Rc représente un atome d'hydrogène ou un groupe (C1-C6)alkyle, (C3-C7)cycloalkyle, phényle ou hétérocycloalkyle de 5 ou 6 chainons, ledit groupe pouvant éventuellement être substitué, ou bien Rb et Rc sont liés entre eux et forment une chaine alkylène, comprenant de 2 à 5 atomes de carbone, éventuellement substituée,
c. on fait réagir dans un solvant ou mélange de solvants, la solution d'un sel de la terre rare correspondante obtenue à l'étape a) et la solution d'un complexe de transfert de charge de formule (I) obtenue à l'étape b) 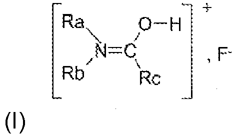
d. on chauffe dans un récipient hermétiquement fermé le mélange obtenu à l'étape c) de manière à obtenir une suspension colloïdale de nanoparticules de fluorure de terre rare,
e. optionnellement, on lave la suspension colloïdale de nanoparticules de fluorure de terre rare, de préférence par au moins un cycle de centrifugation/redispersion dans un solvant approprié jusqu'à ce que le surnageant soit limpide et incolore, étant entendu que le dernier solvant de redispersion est choisi en fonction du solvant d'utilisation de la suspension colloïdale de nanoparticules de fluorure de terre rare.

Un autre objet de l'invention concerne un procédé de préparation d'une suspension colloïdale de nanoparticules de fluorure de terre rare fonctionnalisées dans lequel :
a. on prépare une suspension colloïdale de nanoparticules de fluorure de terre rare comme indiqué précédemment,
b. on greffe à la surface des nanoparticules de fluorure de terre rare une molécule présentant une structure du type A-Sp-Z dans laquelle :
   i. A est une fonction assurant l'accrochage de la molécule à la surface,
   ii. Sp est un groupe espaceur,
   iii. Z est une fonction chimique permettant de modifier le caractère de surface des nanoparticules et/ ou d'insérer lesdites nanoparticules de façon covalente dans un réseau polymérique.
c. optionnellement, on lave la suspension colloïdale de nanoparticules de fluorure de terre rare fonctionnalisées, de préférence par au moins un cycle de centrifugation/redispersion dans un solvant approprié jusqu'à ce que le surnageant soit exempt de molécules fonctionnalisantes libres, étant entendu que le dernier solvant de redispersion est choisi en fonction du solvant d'utilisation de la suspension colloïdale de nanoparticules de fluorure de terre rare fonctionnalisées. Dans un mode de réalisation, le dernier solvant de redispersion est identique au solvant d'utilisation de la suspension, ou au moins miscible avec ce dernier.

Dans un mode de réalisation particulier, on reconcentre la suspension colloïdale de nanoparticules de fluorure de terre rare fonctionnalisées jusqu'à atteindre l'extrait sec désiré, de préférence jusqu'à atteindre un extrait sec de 20%, de 25%, de 30%, de 35%, de 40%, de 45% ou de 50%.

Avantageusement, la terre rare est choisie parmi le lanthane, le cérium, le praséodyme, le néodyme, le prométhium, le samarium, l'europium, le gadolinium, le terbium, le dysprosium, l'holmium, l'erbium, le thulium, l'ytterbium, le lutétium, l'yttrium et le scandium. L'invention a également pour objet une suspension colloïdale de nanoparticules de fluorure de terre rare susceptible d'être obtenue par les procédés précédemment décrits. L'invention a également pour objet l'utilisation d'une suspension colloïdale de nanoparticules de fluorure de terre rare d'une taille D50 inférieure à 300 nm et à une concentration supérieure à 20 % par rapport au poids de la suspension, dans la fabrication d'un matériau de restauration dentaire, et notamment pour le comblement dentaire. L'invention a également pour objet l'utilisation d'une composition comprenant une suspension colloïdale de nanoparticules de fluorure de terre rare susceptible d'être obtenue par les procédés précédemment décrits, dans la fabrication d'un matériau de restauration dentaire, et notamment pour le comblement dentaire. L'invention a également pour objet une composition comprenant au moins une suspension colloïdale de nanoparticules de fluorure de terre rare susceptible d'être obtenue par les procédés précédemment décrits.

L'invention a également pour objet un matériau de restauration dentaire polymérisable comprenant :
- une suspension colloïdale de nanoparticules de fluorure de terre rare susceptible d'être obtenue par les procédés précédemment décrits ou une suspension colloïdale de nanoparticules de fluorure de terre rare susceptible d'être obtenue par les procédés précédemment décrits fonctionnalisées,
- au moins un monomère polymérisable,
- un système d'initiateur de polymérisation radicalaire pour le durcissement thermique ou le photodurcissement dudit composite dentaire polymérisable,
- et des charges.

L'invention a également pour objet un composite dentaire polymérisable comprenant :
(i) de 9 à 29 % en poids de résine organique comprenant au moins un monomère polymérisable et une suspension colloïdale de nanoparticules de fluorure de terre rare rare susceptible d'être obtenue par les procédés précédemment décrits fonctionnalisées ou non,
(ii) de 70% à 90% en poids de charges.
(iii) 1% en poids de composés additionnels comprenant au moins un système initiateur de polymérisation radicalaire pour le durcissement thermique ou le photodurcissement dudit composite dentaire polymérisable.

L'invention a également pour objet un composite dentaire polymérisable comprenant parmi les composés additionnels des inhibiteurs de polymérisation et des pigments selon la revendication 13.

L'invention a également pour objet un matériau de restauration dentaire polymérisable selon la revendication 11 où la polymérisation du matériau de restauration dentaire polymérisable selon la revendication 11 peut être effectuée dans la bouche du patient, de préférence sous rayonnement UV.

L'invention a également pour objet un composite dentaire polymérisable selon la revendication 12 où la polymérisation du composite dentaire polymérisable selon la revendication 12 peut être effectuée dans la bouche du patient, de préférence sous rayonnement UV.

### Description des figures

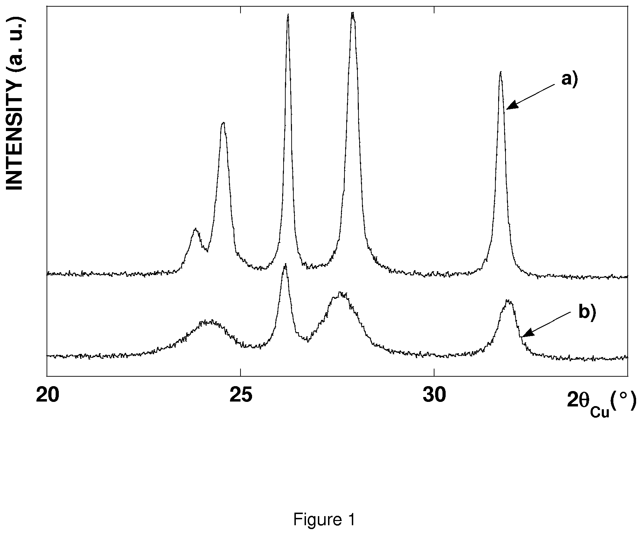

Les figures 1 à 4 illustrent différents aspects de modes de réalisation de l'invention, sans pour autant limiter sa portée.

La figure 1 présente un diagramme de diffraction des Rayons X des nanoparticules de YbF₃ en fonction du procédé de synthèse de ces nanoparticules effectué à 170°C (température du milieu réactionnel) pendant 1h en autoclave (cf. courbe a) ou à 170°C (température du bain) pendant 1h en ballon (cf. courbe b) ; le solvant de synthèse était dans les deux cas un mélange eau / N-méthyl-pyrrolidinone (NMP) en rapport volumique de 1 : 4,3. La principale différence entre ces deux procédés de synthèse réside dans l'emploi d'un autoclave (courbe (a)) ou d'un ballon (courbe (b)) comme réacteur de synthèse. Dans le cadre d'une synthèse de nanoparticules de fluorure de terre rare en ballon, la réaction est effectuée à pression atmosphérique et la température du mélange réactionnel est fixée par la température d'ébullition du solvant ou du mélange de solvants employé. Au regard de la synthèse en ballon, une synthèse de nanoparticules de fluorure de terre rare en autoclave présente deux paramètres ajustables : la pression et la température. En effet, la synthèse des nanoparticules de YbF₃ en autoclave a été effectuée dans un mélange NMP/eau à 170°C à la pression autogène correspondante (3-5 bars).

Le diagramme de diffraction des nanoparticules de YbF₃ obtenues en autoclave (i.e. sous pression) présente des pics de diffraction plus étroits et mieux définis que celui des nanoparticules de YbF₃ obtenues par un procédé en ballon (i.e. à pression atmosphérique) selon le brevet FR 2 946 973. Les nanoparticules de YbF₃ obtenues par un procédé en autoclave présentent ainsi une meilleure cristallinité que les nanoparticules de YbF₃ obtenues par un procédé en ballon.

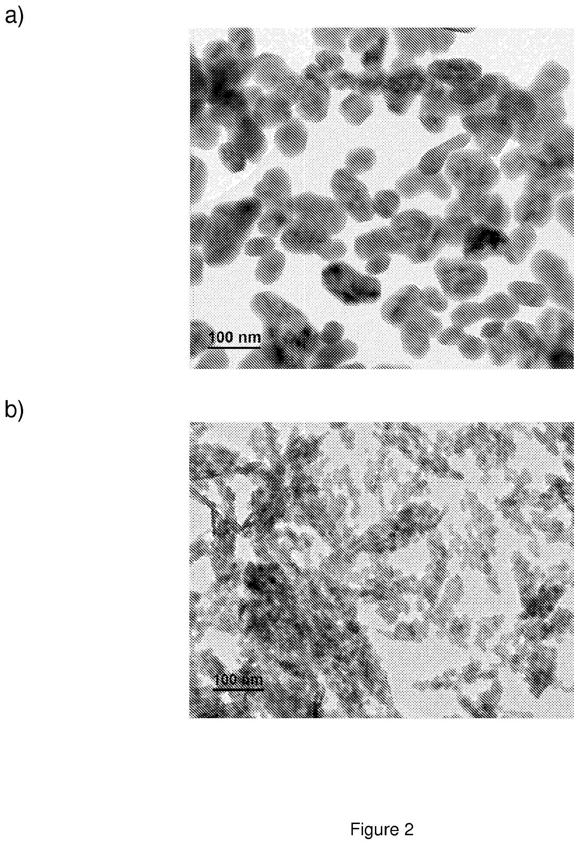

La figure 2 présente des clichés de microscopie électronique des nanoparticules de YbF₃ obtenues par un procédé en autoclave à 170°C (température du milieu réactionnel) pendant 1h (figure 2a)) et par un procédé en ballon à 170°C (température du bain) pendant 1h (figure 2b)). Les nanoparticules de YbF₃ obtenues par un procédé en autoclave sont parfaitement définies et indépendantes les unes des autres, contrairement à celles obtenues par un procédé en ballon qui sont agrégées et de tailles inhomogènes.

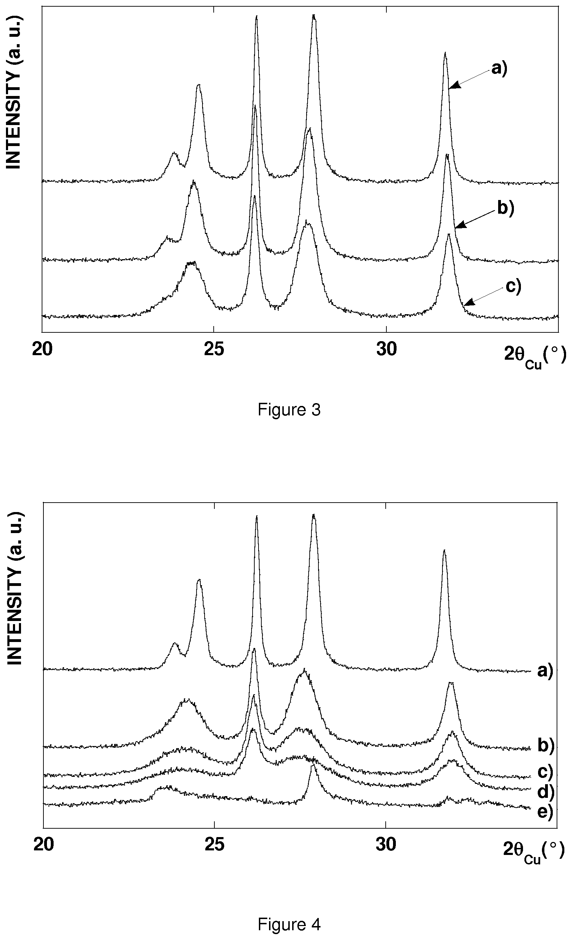

La figure 3 présente des diagrammes de diffraction des Rayons X de nanoparticules de YbF₃ obtenues par un procédé en autoclave à 170°C après 0 min (cf. courbe c)), 30 min (cf. courbe b)) et 60 minutes (cf. courbe a)) de réaction. La cristallinité des nanoparticules de YbF₃ augmente avec le temps de synthèse en autoclave.

La figure 4 présente des diagrammes de diffraction des Rayons X de nanoparticules de YbF₃ obtenues par un procédé en autoclave après 1h de réaction à 50°C (cf. courbe e)), 80°C (cf. courbe d)), 100°C (cf. courbe c)), 120°C (cf. courbe b)) et 170°C (cf. courbe a)). La cristallinité des nanoparticules de YbF₃ est fonction de la température du milieu employée dans l'autoclave pendant la synthèse des particules. La cristallinité des nanoparticules de YbF₃ augmente avec la température du milieu employée dans l'autoclave pendant la synthèse des particules.

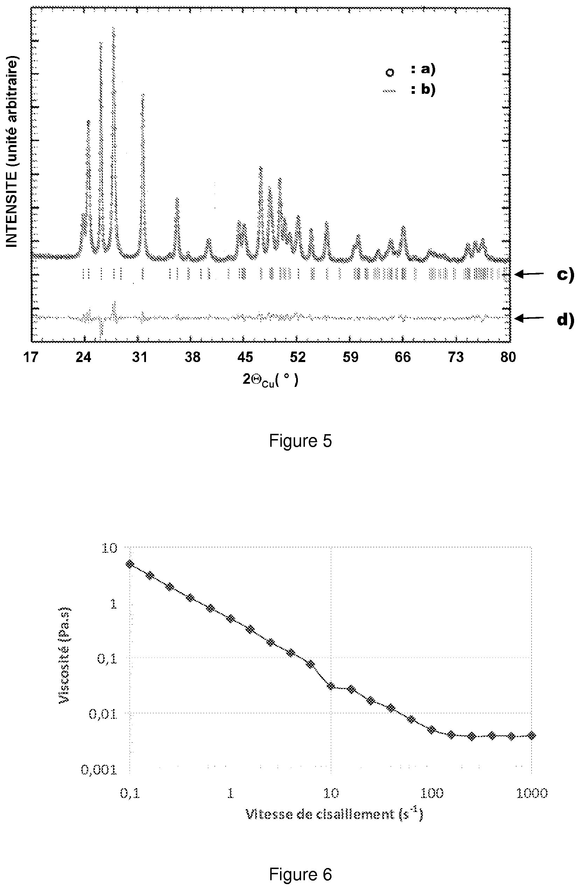

La figure 5 présente l'analyse par diffraction de Rayons X de nanoparticules de fluorure d'ytterbium élaborées en autoclave à 170°C pendant 1h (cf. points expérimentaux en figure 5a). Une simulation du diagramme de diffraction des nanoparticules de fluorure d'ytterbium par la méthode de Rietveld a été effectuée et montrée en figure 5 (cf. courbe b). A partir de cet ajustement les paramètres de maille ont été déterminés : a=6,2004Å, b=6,7917Å et c=4,4633Å. La taille des domaines cohérents de diffraction a également été déterminée, cette taille proche de 30 nm correspond approximativement à la taille des cristallites. La figure 5c) montre les positions des pics de diffraction. La courbe 5d) présente la différence entre les points expérimentaux et l'ajustement issu de la simulation selon la méthode de Rietveld. Le diagramme de diffraction montre ainsi que les nanoparticules sont parfaitement cristallisées et exemptes d'impuretés.

La figure 6 présente l'évolution de la viscosité, à température ambiante, en fonction de la vitesse de cisaillement d'une solution colloïdale dans l'eau de fluorure d'ytterbium présentant un extrait sec de 50% massique.

### Description détaillée

Dans le cadre du présent document, la taille d'une particule est définie par sa plus grande dimension. Par « nanoparticule », on entend toute particule ou objet de taille nanométrique présentant au moins une de ses dimensions inférieure ou égale à 100 nm. Cependant, l'invention est applicable aussi à des particules de taille supérieure à 100 nm, mais de préférence inférieure à 300 nm.

La « taille de particules » ou « taille moyenne de particules » d'une poudre ou d'un ensemble des particules est donnée en D50 et peut notamment être mesurée par diffusion dynamique de la lumière.

La présente invention concerne des suspensions colloïdales de fluorure de terre rare sous forme de nanoparticules, un procédé de synthèse de nanoparticules de fluorure de terre rare, la fonctionnalisation de ces nanoparticules et leurs utilisations comme nanocharges dans des compositions, notamment dans des compositions dentaires, de préférences dans des matériaux de restauration dentaires polymérisables tels que des composites dentaires polymérisables.

La taille des nanoparticules de fluorure de terre rare présentes dans les suspensions selon l'invention influe sur l'aspect final des composites dentaires dans lesquels elles sont incorporées. En effet, à teneur massique en fluorure de terre rare égale, la translucidité des matériaux et composites dentaires augmente lorsque la taille des nanoparticules de fluorure de terre contenues dans la suspension selon l'invention diminue. Au-delà du côté esthétique, une translucidité améliorée permet d'augmenter la profondeur de polymérisation.

Les particules selon l'invention sont stables au stockage, conçues pour un stockage et une utilisation à des extraits secs supérieurs à 20% massique, de préférence supérieurs à 23% massique et encore plus préférentiellement supérieurs à 25% massique.

### Synthèse de nanoparticules de fluorure de terre rare en autoclave

L'invention concerne un procédé de préparation de nanoparticules composées, au moins en partie, d'un fluorure de terre rare. Ce procédé est caractérisé en ce que ledit fluorure est obtenu, en solution, à partir d'un sel de la terre rare correspondante et d'un complexe de transfert de charge obtenu par la réaction d'un amide de formule II avec une source d'ions fluorure telle que de l'acide fluorhydrique: 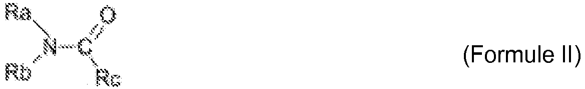 où :
- Ra et Rb, identiques ou différents, représentent, chacun indépendamment l'un de l'autre, un groupe (C₁-C₆)alkyle, (C₃-C₇)cycloalkyle, phényle ou hétérocycloalkyle de 5 ou 6 chaînons, ledit groupe pouvant éventuellement être substitué, ou bien Ra et Rb sont liés entre eux et forment une chaine alkylène, comprenant de 2 à 6 atomes de carbone, éventuellement substituée, et
- Rc représente un atome d'hydrogène ou un groupe (C₁-C₆)alkyle, (C₃-C₇)cycloalkyle, phényle ou hétérocycloalkyle de 5 ou 6 chaînons, ledit groupe pouvant éventuellement être substitué, ou bien Rb et Rc sont liés entre eux et forment une chaine alkylène, comprenant de 2 à 5 atomes de carbone, éventuellement substituée.

Par (C₁-C₆)alkyle, on entend un groupe hydrocarboné saturé, linéaire ou ramifié, comprenant de 1 à 6 atomes de carbone, tels que les groupes méthyle, éthyle, iso-propyle, n-propyle, sec-butyle, tett-butyle, n-butyle, n-pentyle, n- hexyle.

Par chaine alkylène comprenant de 2 à 6 atomes de carbone, on entend une chaine -(Ch₂)ₚ- avec p = 2, 3, 4, 5 ou 6.

Par (C₃-C₇)cycloalkyle, on entend un groupe hydrocarboné saturé cyclique, comprenant de 3 à 7 atomes de carbone.

Par hétérocycloalkyle, on entend un groupe cycloalkyle tel que défini ci- dessus dans lequel l'un au moins des atomes de carbones a été remplacé par un hétéroatome, du type N ou S, et notamment un groupe pyrrolidine ou pipéridine.

Par source d'ions fluorure F⁻, on entend tout composé chimique dont sa dissociation fournit des ions fluorure, tel que l'acide fluorhydrique (HF), le fluorure de tétrabutylammonium, le trihydrofluorure de triéthylamine ou l'acide fluorosilicique. Avantageusement, la source d'ions fluorure employée est l'acide fluorhydrique.

Dans le cas où un des groupes (C₁-C₆)alkyle, (C₃-C₇)cycloalkyle, ou phényle, sera substitué, il pourra être substitué par un ou plusieurs substituants, notamment choisis parmi les halogènes (F, Cl, Br, I), les groupes (C₁-C₆) alkyle, phényle, hydroxy (-OH), (C₁-C₆) alkylhydroxy (-(C₁-C₆)alkylOH). Néanmoins, de préférence, ces groupes seront non substitués, leur préparation étant plus aisée dans ce cas.

Bien entendu, dans le complexe de transfert de charge de formule (I), lorsque Ra et Rb seront liés entre eux pour former une chaine alkylène, Rc représentera un atome d'hydrogène ou un groupe (C₁-C₆)alkyle, (C₃-C₇)cycloalkyle, phényle ou hétérocycloalkyle de 5 ou 6 chaînons, éventuellement substitué et Rc ne pourra pas être lié à Rb.

De même, lorsque Rb et Rc seront liés entre eux pour former une chaîne alkylène, Ra représentera, un groupe (C₁-C₆)alkyle, (C₃-C₇)cycloalkyle, phényle ou hétérocycloalkyle à 5 ou 6 chaînons, éventuellement substitué, et Ra ne pourra pas être lié à Rb.

Dans le cadre du procédé selon l'invention, le complexe de transfert de charge est préparé séparément avant toute réaction avec un sel de terre rare.

Selon l'invention, la réaction entre le sel de terre rare et le complexe de transfert de charge est réalisée dans un mélange d'un premier solvant (appelé ici « solvant 1 »), d'un amide, et éventuellement d'un deuxième solvant (appelé ici « solvant 2 »), le solvant 2 étant différent de l'amide. Le solvant 1 doit être choisi pour permettre de dissoudre le sel de terre rare utilisé. Le sel de terre rare utilisé est, par exemple, choisi parmi les chlorures, nitrates et alkoxydes ; les chlorures sont préférés, sachant que les nitrates présentent un risque d'explosion, et sachant que les alkoxydes sont chers et difficilement manipulables à cause de leur réactivité en présence d'humidité. La terre rare, quant à elle, peut notamment être choisie parmi le lanthane, le cérium, le praséodyme, le néodyme, le prométhium, le samarium, l'europium, le gadolinium, le terbium, le dysprosium, l'holmium, l'erbium, le thulium, l'ytterbium, le lutécium, l'yttrium et le scandium. Avantageusement, la terre rare employée est de l'ytterbium.

Le solvant 2 est utilisé pour dissoudre l'amide si ce dernier n'est pas liquide.

Le solvant du mélange utilisé pour la réaction entre le sel de terre rare et le complexe de transfert de charge peut correspondre au solvant utilisé pour préparer le complexe de transfert de charge. De préférence, le solvant utilisé pour la réaction entre le sel de terre rare et le complexe de transfert de charge est un solvant non toxique tel que l'eau.

Par exemple, le complexe de transfert de charge peut être obtenu par réaction de HF, comme source d'ions fluorure, et de diméthylformamide, conduisant à la formation d'un complexe de transfert de charge dans lequel Ra=Rb=méthyle et Rc=H. Le complexe de transfert de charge peut également être obtenu par réaction de HF et de diméthylacétamide, conduisant à la formation d'un complexe de transfert de charge dans lequel Ra=Rb=Rc=méthyle. Selon une autre alternative, le complexe de transfert de charge peut être obtenu par réaction de HF et de N-méthylpyrrolidinone, conduisant à la formation d'un complexe de transfert de charge dans lequel Ra= méthyle et -Rc-Rb-=-(CH₂)₃-. La préparation du complexe de transfert de charge pourra être effectuée, notamment à température ambiante et sous pression atmosphérique, et ce en l'absence de solvant additionnel, le diméthylformamide, le diméthylacétamide ou la N-méthylpyrrolidinone jouant le rôle à la fois de réactif et de solvant. De préférence, par la suite, le diméthylformamide, le diméthylacétamide ou la N-méthylpyrrolidinone joue également le rôle de solvant pour la réaction entre le sel de terre rare et le complexe de transfert de charge. Une partie du solvant sert à stabiliser la suspension colloïdale qui va être obtenue, par effet de complexation à la surface des nanoparticules. De façon avantageuse, la réaction entre le sel de terre rare et le complexe de transfert de charge est réalisée dans un mélange de solvants comprenant le solvant 1, l'amide si ce dernier joue le rôle de solvant et éventuellement le solvant 2. Avantageusement, le mélange de solvant contient un solvant protique, tel que l'eau, l'éthanol ou l'isopropanol. Avantageusement, le solvant protique est l'eau.

Dans le procédé selon l'invention, on fait réagir une solution d'un complexe à transfert de charge de formule (II) préalablement formé, avec un sel de la terre rare. L'anion du complexe à transfert de charges F⁻ va réagir, en solution, avec les ions de la terre rare sélectionnée du type RE³⁺ (où « RE » symbolise un atome de terre rare (« Rare Earth » en anglais).

Le complexe de transfert de charge est obtenu par la réaction d'une source d'ions fluorure F⁻ telle que de l'acide fluorhydrique avec un amide de formule II présent en excès dans le milieu réactionnel. Avantageusement, le ratio molaire amide de formule II / F⁻ est supérieur ou égal à 2, de préférence compris entre 2 et 57, plus préférentiellement entre 5 et 6 et encore plus préférentiellement d'environ 5,6. L'amide de formule II peut avantageusement jouer le rôle à la fois de réactif et de solvant. Etant donné que l'amide II peut jouer le rôle de solvant et que la quantité d'ions fluorure F⁻ dépend directement de la quantité de sel de terre rare tel que le sel d'ytterbium, le ratio molaire amide de formule II / F⁻ est affecté lorsque la concentration en sel de terre rare dans le milieu de synthèse est modifié. Lorsque la synthèse est effectuée à une concentration plus élevée en sel de terre rare dans le milieu réactionnel, le ratio molaire amide de formule II / F⁻ diminue. Cependant, pour des ratios molaires amide de formule II / F⁻ inférieurs à 2, le sel de terre rare, tel que le sel d'ytterbium, n'est pas soluble dans le milieu réactionnel.

De même une synthèse en conditions plus diluées en sel d'Ytterbium induit une augmentation du ratio molaire amide de formule II / F⁻. Le complexe de transfert de charge peut être obtenu à des ratios molaires amide de formule II / F⁻ supérieurs à 57. Cependant, plus le milieu réactionnel contiendra d'amide de formule II, plus le milieu réactionnel sera dilué entrainant une consommation inutile d'amide de formule II et donc un surcoût inutile.

La réaction entre le sel de terre rare préalablement solubilisé dans le solvant 1 et le complexe de transfert de charge est effectuée de manière à obtenir un ratio molaire RE/F⁻inférieur à 1, de préférence d'environ 0,37. Avantageusement, l'ion fluorure est le réactif limitant de cette réaction. L'augmentation du ratio RE/F⁻ diminue légèrement la taille des nanoparticules obtenues. Cependant utiliser un trop grand excès de sel de terre rare induit un surcoût. Pour un ratio RE/F⁻ constant, la modification de la concentration en sel de terre rare, notamment en sel d'ytterbium, dans le milieu réactionnel n'a pas d'impact sur les caractéristiques des nanoparticules obtenues.

Le ratio solvant 1/ amide de formule II est compris entre 0,5 et 50, de préférence entre 0,75 et 6 et encore plus préférentiellement égale à environ 1,03. L'augmentation du ratio solvant 1 / amide de formule II permet d'accroître la taille des nanoparticules de fluorure de terre rare.

Le milieu réactionnel est ensuite introduit dans un autoclave muni d'un moyen de mesure de la température et d'un système d'agitation. L'autoclave est ensuite hermétiquement fermé et disposé dans un manteau chauffant.

On entend ici par autoclave un récipient hermétiquement fermé, c'est-à-dire un récipient qui ne laisse pas échapper une surpression qui se crée lors de l'échauffement d'un volume de gaz ou de liquide contenu dans ledit récipient, même au-delà du point d'ébullition normal dudit liquide.

Lorsque la température de l'autoclave atteint la température de consigne comprise entre 20 et 200°C, de préférence entre 70 et 190°C, et avantageusement à environ 170°C, le mélange est maintenu à cette température de consigne pendant un temps t sous agitation.

Avantageusement, le mélange est maintenu dans l'autoclave à la température de consigne (170°C) pendant un temps compris entre 1 minute et 6 heures, de préférence entre 30 minutes et 2 heures, et encore plus préférentiellement pendant environ 1 heure.

Les avantages du procédé en autoclave sont présentés en figures 1 et 2 au regard du procédé divulgué dans le brevet FR 2 946 973 où les nanoparticules de fluorure de terre rare sont synthétisées en ballon. La principale différence entre ces deux procédés de synthèse réside dans l'emploi d'un autoclave (courbe (a)) ou d'un ballon (courbe (b)) comme réacteur de synthèse. Dans le cadre d'une synthèse de nanoparticules de fluorure de terre rare en ballon, la réaction est effectuée à pression atmosphérique et la température du mélange réactionnel est fixée par la température d'ébullition du solvant ou du mélange de solvants employé. Par rapport à la synthèse en ballon, une synthèse en autoclave présente deux paramètres ajustables : la pression et la température. En effet, la synthèse des nanoparticules de YbF₃ en autoclave a été effectuée dans un mélange eau / NMP à 170°C à la pression autogène correspondante (3-5 bars). Les particules obtenues par un procédé en ballon et un procédé en autoclave à 170°C sous pression pendant 1h présentent une cristallinité et une morphologie très différentes. Les nanoparticules obtenues par un procédé en autoclave sont mieux cristallisées et plus petites que celles synthétisées en ballon.

Dans le cadre d'une synthèse de nanoparticules de fluorure de terre rare en autoclave, les figures 3 et 4 enseignent que la température et le temps de chauffage dans l'autoclave influencent aussi la cristallinité des particules synthétisées.

Les nanoparticules de fluorure d'ytterbium élaborées en autoclave à 170°C sous pression pendant 1h ont été analysées par diffraction de rayons X (cf. figure 5a). Une simulation du diagramme de diffraction des nanoparticules de fluorure d'ytterbium par la méthode de Rietveld a été effectuée et montrée en figure 5b). Cette modélisation permet de déterminer les paramètres de maille du fluorure d'Ytterbium et démontre que les nanoparticules de fluorure d'ytterbium élaborées en autoclave sont parfaitement cristallisées et exemptes d'impuretés.

Ainsi avantageusement, afin d'obtenir des nanoparticules de fluorure de terre rare cristallisées, les conditions de synthèses sont optimisées : la température de consigne de l'autoclave contenant le milieu réactionnel est fixée à environ 170°C et le mélange réactionnel est maintenu à cette température de consigne pendant environ une heure sous agitation.

L'autoclave est ensuite refroidi (vitesse maximale 10°C/min) avant ouverture et extraction du milieu réactionnel.

Le milieu réactionnel est ensuite introduit dans une solution d'acétone induisant la formation immédiate d'un précipité blanc des nanoparticules de fluorure de terre rare. De préférence, le solvant utilisé pour précipiter les nanoparticules de fluorure de terre rare est un solvant non toxique.

Après quelques heures (typiquement 6 heures), le solide a précipité et le surnageant est ensuite éliminé par tout moyen approprié. La suspension résultante est centrifugée. Le culot obtenu est ensuite redispersé dans du méthanol. De préférence, le solvant utilisé pour redisperser le culot est un solvant non toxique tel que l'eau.

La solution obtenue est ensuite soumise à des cycles de centrifugation / redispersion de manière à purifier les nanoparticules de fluorure de terre rare. Au moins un cycle, de préférence, deux cycles de centrifugation / redispersion sont effectués à des accélérations centrifuges comprises entre 648 g et 16 211 g pendant 5 à 30 minutes. Ce(s) cycle(s) de centrifugation / redispersion permettent de séparer les fluorures de terre rare des composés n'ayant pas réagi et des sous-produits de réaction présents dans les surnageants.

La redispersion est effectuée en introduisant une certaine quantité (4 mL de méthanol/ g de REF₃) d'un solvant sur le culot et par l'emploi de tout moyen de dispersion tel que le vortex ou un bain à ultrasons.

Après ces cycles de centrifugation / redispersion, le culot est redispersé dans l'eau et la suspension colloïdale obtenue (de 40 à 60 % massique) est soumise aux ultrasons pendant 1 à 2 min par l'emploi d'une sonde à ultrasons (Hielscher, UP400S).

Avantageusement, afin de purifier ces nanoparticules de fluorure de terre rare, la suspension colloïdale précédente est introduite de nouveau dans une solution d'acétone induisant la formation d'un précipité blanc qui subira de nouveau des cycles de centrifugation/ redispersion.

L'extrait sec de la suspension colloïdale finale est ensuite mesuré sur un échantillon de la suspension. La viscosité, à température ambiante, d'une suspension colloïdale dans l'eau de fluorure d'ytterbium présentant un extrait sec de 50% massique a été mesurée et présentée en figure 6 en fonction de la vitesse de cisaillement de ladite suspension.

Ce procédé de synthèse en autoclave qui vient d'être décrit peut être mis en oeuvre avec ses domaines préférentiels notamment pour la synthèse de nanoparticules de fluorure d'ytterbium.

### Fonctionnalisation des nanoparticules de fluorure de terre rare

Les nanoparticules de fluorure de terre rare précédemment obtenues peuvent ensuite être fonctionnalisées, avec des molécules organiques, selon des méthodes connues de l'homme du métier. La fonctionnalisation consiste à greffer à la surface des nanoparticules une molécule présentant une structure du type A- Sp-Z dans laquelle :
- A est une fonction assurant l'accrochage de la molécule à la surface,
- Sp est un groupe espaceur qui participe à la balance hydrophile / hydrophobe et à la dispersion des particules,
- Z une fonction chimique réactive ou non qui participe à la balance hydrophile / hydrophobe, à la dispersion des particules et éventuellement à leur interaction avec leur milieu de dispersion (insertion covalente dans un réseau polymère).

Comme groupe A, une fonction complexante des cations de surface des nanoparticules peut être utilisée (telle que: phosphate, phosphonate, carboxylate, dithiophosphate, dithiophosphonate). La force de complexation de ce groupement peut être renforcée par l'utilisation de fonctions multiples (telles que : polyacide, polyphosphate).

Comme groupe espaceur Sp, on peut utiliser n'importe quel espaceur connu de l'homme du métier, par exemple une chaîne alkyl linéaire ou branchée ou un groupe aromatique ou une combinaison de ces groupes, pouvant être interrompus par un ou plusieurs hétéro atomes choisis parmi O, N, S, P.

Comme fonction chimique Z on pourra utiliser n'importe quelle fonction chimique connue de l'homme de l'art, de préférence les fonctions méthacrylate, acrylate, glycidyl, alcool, methoxy. Par exemple, une fonctionnalisation de la surface du fluorure de terre rare obtenu peut être réalisée avec une molécule organique porteuse d'une fonction biphosphonate.

De préférence, les molécules fonctionnalisantes A-Sp-Z sont choisies parmi le :
- methoxy-PEG-C6-phosphonate (aussi connu sous le nom P-7,10,13,16-tetraoxaheptadec-1-yl-phosphonic acid),
- l'hydroxy-PEG-C6-phosphonate (aussi connu sous le nom P-[6-[2-[2-(2-hydroxyethoxy)ethoxy]ethoxy]hexyl]phosphonic acid),
- le phosphonate-dimethacrylate (aussi connu sous le nom 2,3-di-(methacryloyloxy)-propyl-1-phosphonic acid),
- le bisphosphonate-dimethacrylate (aussi connu sous le nom 2,3-di-(methacryloyloxy)-propyl-1-bisphosphonic acid)
- et le bisphosphonate-methacrylate-O-benzyl (aussi connu sous le nom 3-O-benzyloxy-2-methacryloyloxy-propyl-1-bisphosphonic acid).

Selon un mode de réalisation avantageux, une suspension colloïdale de fluorure de terre rare REF₃ est ajoutée à une solution d'A-Sp-Z de manière à obtenir un ratio molaire REF₃/A-Sp-Z compris entre 1 et 100, de préférence entre 5 et 50, et plus préférentiellement entre 10 et 30. Au-delà d'un ratio molaire REF₃/A-Sp-Z de 50, la molécule A-Sp-Z risque de ne pas être introduite en quantité suffisante pour assurer la stabilité des particules fonctionnalisées ; cela dépend aussi de la taille de particules. Pour un ratio molaire REF₃/A-Sp-Z inférieur à 5, la fonctionnalisation des nanoparticules de REF₃ par la molécule A-Sp-Z risque d'induire un encombrement stérique tel, que les particules de fluorure de terre rare ne peuvent pas être plus fonctionnalisées ; cela dépend aussi de la taille de particules. L'utilisation d'une quantité d'A-Sp-Z supérieure lors de la fonctionnalisation entrainerait une consommation inutile d'A-Sp-Z.

Avantageusement, le fluorure de terre rare REF₃ est le YbF₃.

Une suspension colloïdale de fluorure de terre rare à une concentration massique comprise entre 1% et 30%, de préférence entre 10% et 20%, et encore plus préférentiellement à 15% est utilisée pour réaliser la fonctionnalisation des particules de fluorure de terre rare. A forte concentration, il peut y avoir un risque de pontage et un manque d'accessibilité de la surface à fonctionnaliser (risque de précipitation de particules non ou mal fonctionnalisées). De préférence, les fluorures de terre rare sont dispersés dans un solvant non toxique, tel que l'eau ou l'éthanol.

Cette réaction peut être réalisée dans tous solvants appropriés, de préférence dans des solvants non-toxiques, permettant de solubiliser la molécule A-Sp-Z.

En fonction de la molécule A-Sp-Z les conditions de fonctionnalisation peuvent être optimisées (température, durée, solvants...). Après avoir ajouté une solution d'A-Sp-Z à une suspension colloïdale de fluorure de terre rare, le milieu réactionnel est laissé sous agitation pendant quelques heures, de manière à ce qu'au moins une partie, de préférence la totalité des molécules A-Sp-Z puisse être greffée à la surface des particules de fluorure de terre rare. La fonctionnalisation peut être réalisée sous chauffage, de préférence à une température comprise entre 40°C et 70°C. La température du milieu réactionnel doit être adaptée au choix de la molécule fonctionnalisante.

Avantageusement, les nanoparticules ainsi fonctionnalisées sont ensuite purifiées par des cycles de centrifugations et redispersions successives et /ou par filtration tangentielle. Dans un mode de réalisation, la suspension colloïdale de particules de fluorure de terre rare fonctionnalisées est centrifugée pendant un temps t compris entre 5 et 30 minutes, entre 648 et 16 211 g, de manière à séparer les particules fonctionnalisées des molécules n'ayant pas réagi présentes dans le surnageant. Le surplus de molécules A-Sp-Z, n'ayant pas réagi et toujours présentes dans le surnageant, est désigné ci-après « molécules fonctionnalisantes libres ». Après centrifugation, le surnageant est éliminé. Le culot comprenant les particules fonctionnalisées est redispersé dans le solvant. Cette redispersion peut être effectuée par tout moyen, notamment par l'emploi d'un bain à ultrasons ou l'emploi d'une sonde à ultrasons (Hielscher, UP400S), ou encore sous agitation magnétique et/ou manuelle. Plusieurs moyens de redispersions peuvent être combinés pour permettre une bonne redispersion des particules dans le solvant.

Plusieurs cycles de centrifugations et redispersions successives peuvent être effectués de manière à éliminer les molécules n'ayant pas réagi. De préférence on réalise au moins un, encore plus préférentiellement au moins deux cycles de centrifugations et redispersions successifs.

La redispersion des particules de fluorure de terre rare fonctionnalisées peut être réalisée dans un solvant non toxique, notamment dans l'eau.

La redispersion peut être réalisée en plusieurs étapes. Après centrifugation, le surnageant est extrait. Le solvant ajouté sur le culot permet de redisperser au moins une partie du culot. La solution colloïdale issue de la mise en solution d'une partie du culot est extraite et du solvant est à nouveau ajouté sur le culot restant. Cette opération est alors répétée jusqu'à la redispersion complète du culot. Les fractions de la solution colloïdale récupérées sont ensuite rassemblées.

Après redispersion des particules de fluorure de terre rare fonctionnalisées, la suspension peut être reconcentrée jusqu'à atteindre l'extrait sec souhaité, par tout moyen approprié, et notamment à l'aide d'un évaporateur rotatif.

D'autres solvants, notamment des monomères, peuvent être employés pour redisperser les nanoparticules de fluorure de terre rare, tel que le triéthylène glycol diméthacrylate (TEG-DMA), le 2-hydroxyéthyl méthacrylate (HEMA), un mélange de bisphénol A glycerolate diméthacrylate (Bis-GMA) /TEG-DMA, un mélange de Bis-GMA / bisphénol A glycol éthoxylé diméthacrylate (BisEMA) / TEG-DMA ou un mélange de Bis GMA / 1,10-décanediol diméthacrylate (D3MA). Les proportions respectives de ces solvants utilisés en mélange peuvent varier en fonction de l'utilisation finale.

Le tableau 1 ci-après présente des couples molécule fonctionnalisante / solvant de dispersion pouvant être employés ainsi que les extraits secs des suspensions de nanoparticules de fluorure de terre rare fonctionnalisées obtenues après fonctionnalisation, purification et redispersion selon l'invention.

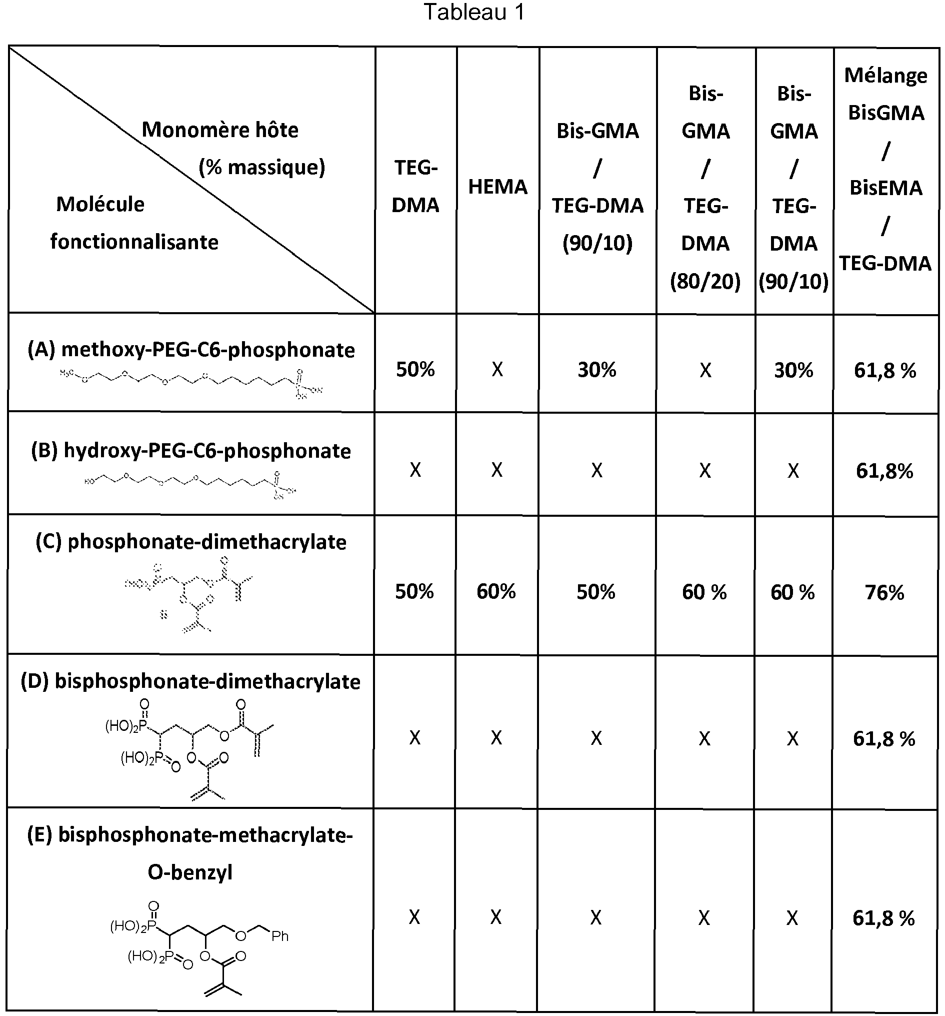

Ce procédé de fonctionnalisation qui vient d'être décrit peut être mis en œuvre avec ses domaines préférentiels notamment pour la synthèse de nanoparticules de fluorure d'ytterbium.

### Fabrication d'un composite dentaire (matériau de restauration dentaire)

Un composite dentaire comprend une matrice organique ou matrice résineuse dans laquelle sont dispersées des charges inorganiques.

La matrice organique est généralement composée d'un mélange de monomères méthacryliques. Dans ce mélange de monomères, tous les monomères participent, lors de leur polymérisation, à la réalisation du réseau polymérique. Le choix des monomères impacte les propriétés d'une part du composite et d'autre part de la résine résultant de la polymérisation du composite. Par exemple, le Bis-GMA / TEG-DMA est un mélange de monomères connus où l'ajout de TEG-DMA permet de diminuer la viscosité du mélange Bis-GMA / TEG-DMA.

Les charges inorganiques sont dispersées dans la matrice organique et jouent le rôle de renfort et d'agent de contraste aux Rayons X. L'introduction de ces charges dans la matrice permet d'améliorer les propriétés mécaniques, de moduler la viscosité et de faciliter la manipulation du composite dentaire. Plusieurs types de charges inorganiques peuvent être employés tels que des macrocharges (de 1 à 10 µm) composées de particules de verre ou de quartz, et des microcharges (de 7 à 40 nm) composées de particules de silice. Les macrocharges améliorent les propriétés mécaniques du composite dentaire. Les microcharges sont des composés qui permettent d'améliorer l'état de surface du composite dentaire.

Le composite dentaire comprend aussi :
- des initiateurs de polymérisation qui activent la polymérisation des monomères afin d'obtenir un réseau polymérique organique en trois dimensions (résine). Ces initiateurs de polymérisation permettent le durcissement thermique ou le photodurcissement du composite dentaire polymérisable. A titre d'exemples, la camphorquinone peut être employée comme agent de photopolymérisation, les amines en présence de peroxyde de benzoyle peuvent être employées comme agent de chemopolymérisation,
- des inhibiteurs de polymérisation qui empêchent la polymérisation des monomères avant toute utilisation du composite, telle que l'hydroquinone, le BHT (2, 4, 6-tritertiary-butyl phénol),
- des pigments tels que de l'oxyde de fer ou de l'oxyde de titane.

Un composite dentaire typique selon l'invention est composé de 9 à 29% massique de résine organique, de 70 à 90% massique de charges inorganiques comprenant des macrocharges et des microcharges et 1% massique de composés additionnels comprenant des initiateurs de polymérisation, des inhibiteurs de polymérisation et des pigments.

Selon un mode de réalisation de l'invention, on utilise une suspension colloïdale à 50% massique de particules de fluorure de terre rare précédemment fonctionnalisées dispersée dans un mélange de monomères (par exemple le Bis-GMA / TEG-DMA). La présence de solvant dispersant facilite l'homogénéisation des particules de fluorures de terre rare dans le mélange de monomères. Après homogénéisation, au moins une partie du solvant est éliminée. A environ 80 à 95% massiques de cette suspension colloïdale on ajoute des charges sous forme de poudre. Après homogénéisation de ce mélange, une pâte est obtenue. On ajoute ensuite des initiateurs de polymérisation (permettant la polymérisation thermique ou la photopolymérisation) et des inhibiteurs de polymérisation qui empêchent la polymérisation des monomères avant toute utilisation du composite. On peut ajouter également des pigments.

Lesdites charges contribuent à diminuer le retrait et améliorent les propriétés mécaniques et le taux de remplissage des cavités dentaires. Cette composition dentaire peut ensuite être utilisée par le praticien pour obturer et reconstituer les dents. Après comblement de la cavité dentaire, la polymérisation de la composition dentaire peut être effectuée dans la bouche du patient, de préférence sous rayonnement UV.

Selon l'état de la technique, on incorpore les charges radio-opacifiantes, telles que les particules de fluorures de terre rare, sous leur forme pulvérulente dans les préparations polymérisables pour composites dentaires ou matériau de restauration dentaire. Cela présente l'inconvénient suivant : lors du séchage des particules sous forme de poudre, les particules s'agglomèrent et forment des agrégats de dimensions largement supérieurs à leur taille initiale. De plus le mélange homogène d'une poudre avec un liquide est beaucoup plus difficile à obtenir que lorsqu'on mélange deux solutions comme cela est le cas dans le cadre de la présente invention. Ainsi, l'emploi d'une suspension colloïdale selon l'invention dans la formulation d'un composite dentaire à la place d'une poudre à base de particules de fluorure de terre rare améliore la consistance, la translucidité, la résistance à l'abrasion et permet d'accroitre la radio-opacité ainsi que la contrainte de rupture en flexion du composite.

### Exemples

L'invention est illustrée ci-dessous par des exemples qui cependant ne limitent pas l'invention. Ces exemples portent sur des suspensions colloïdales de nanoparticules de fluorure de terre rare, le procédé de synthèse de nanoparticules de REF₃, la fonctionnalisation de ces nanoparticules et leurs utilisations comme (nano-) charges dans des composites dentaires polymérisables ou dans des matériaux de restauration dentaire.

### Exemple 1 : Synthèse de nanoparticules de fluorure d'ytterbium en autoclave

141,6 g de YbCl₃, 6H₂O ont été introduits dans un bécher et mélangé à 80 mL d'eau sous agitation de manière à dissoudre le chlorure d'ytterbium hydraté.
538,8 mL de NMP ont été prélevés à l'éprouvette.
40,02 g de HF à ~ 50% (34,5 mL) sont été introduits dans un bécher en téflon servant de réacteur de synthèse. La NMP a ensuite été versé dans le bécher en téflon permettant la formation du complexe à transfert de charge (cations amidium).

Sous agitation énergique, la solution aqueuse d'YbCl₃ a été ajoutée à la solution de NMP / HF dans le bécher en téflon.

Le mélange a ensuite été laissé sous agitation pendant 15 min.

Le bécher en téflon contenant le mélange a ensuite été placé dans l'autoclave muni d'une sonde de température. L'autoclave a ensuite été fermé puis placé sous agitation dans une étuve préalablement chauffée.

Lorsque la température de l'autoclave a atteint la température de consigne de 170°C soit environ 1h30 après introduction dans l'étuve, le mélange a été maintenu à 170°C pendant 1h sous agitation.

L'autoclave a ensuite été refroidi (refroidissement naturel) avant ouverture et extraction du milieu réactionnel.

Le milieu réactionnel a ensuite été versé dans un bécher contenant 700 mL d'acétone. Une formation immédiate d'un précipité blanc décantant lentement a été observée.

Après quelques heures (6h), le solide a précipité et le surnageant limpide, plutôt jaune, a ensuite été éliminé à la pompe péristaltique. La suspension résultante a été centrifugée 10 minutes à 2594 g. Les culots blancs obtenus ont ensuite été redispersés dans 250 mL de méthanol au total.

Une centrifugation à 2594 g pendant 10 minutes a été réalisée de manière à pouvoir obtenir un solide blanc et facilement extraire le surnageant légèrement jaune.

Les culots solides ont été redispersés en solution dans 250 mL de MeOH au total par l'emploi de vortex et d'un bain à ultrasons.

Une centrifugation à 5836 g pendant 10 minutes a été réalisée de manière à pouvoir facilement extraire le surnageant limpide et ainsi à obtenir un culot solide blanc pouvant ultérieurement être redispersé.

Les culots ont ensuite été redispersés par ajout de 60mL d'eau au total. Les nanoparticules ont pu repasser très facilement en solution par l'emploi d'une agitation manuelle, d'un bain à ultrason et d'un vortex. La suspension colloïdale a ensuite été soumise aux ultrasons pendant 1 à 2 min par l'emploi d'une sonde à ultrasons (Hielscher, UP400S).

La solution a pu être utilisée telle quelle ou les nanoparticules ont pu être dispersées dans l'éthanol suivant la procédure décrite ci-après. 90 mL de suspension colloïdale aqueuse à 50% massique ont été introduits dans 600 mL d'acétone, ceci induisant la précipitation des nanoparticules de fluorure d'Ytterbium.

La suspension a été maintenue 5 minutes sous agitation afin d'homogénéiser le milieu puis laissée sans agitation pendant 6 heures de manière à favoriser la sédimentation des nanoparticules de fluorure d'Ytterbium. Après sédimentation, le surnageant limpide et incolore a été éliminé à la pompe péristaltique.

La suspension obtenue a ensuite été centrifugée pendant 5 minutes à 648 g. Après centrifugation, le surnageant a été éliminé et 550 mL d'EtOH ont été introduit sur le culot. La redispersion du culot a été effectuée sous agitation manuelle, par l'emploi d'un bain à ultrasons (de 1 à 10 cycles de 5 minutes), sous vortex puis la dispersion a été améliorée à la sonde aux ultrasons (Hielscher, UP400S).

L'extrait sec de la suspension de nanoparticules de fluorure d'ytterbium a été de 14% en poids.

### Exemple 2 : Synthèse de nanoparticules de fluorure d'ytterbium fonctionnalisées par du phosphonate-diméthacrylate

La synthèse des nanoparticules de fluorure d'ytterbium a été effectuée selon un procédé comparable à celui décrit à l'exemple 1. Une suspension de nanoparticules de fluorure d'ytterbium à 15% massique dans l'eau a été utilisée pour l'étape de fonctionnalisation.

Une solution éthanolique de phosphonate-diméthacrylate a été réalisée à une concentration de 0.2% massique. 100 µL d'une solution éthanolique de MEHQ a été ajoutée à 10 mL de la solution éthanolique de phosphonate-diméthacrylate.

8,8 g de la solution éthanolique de phosphonate-diméthacrylate précédente ont été prélevés et ajoutée au goutte-à-goutte sous très vive agitation dans 267g d'une solution de nanoparticules de fluorure d'ytterbium à 15% massique.

La suspension colloïdale blanchit et a tendance à prendre en masse.

La solution a ensuite été chauffée à 50°C pendant 1h sous vive agitation de manière à parfaire la fonctionnalisation.

La solution a ensuite été laissée à température ambiante sous agitation pendant 15 min.

La solution obtenue a ensuite été répartie dans 2 pots de centrifugation. Une première centrifugation à 2594 g pendant 10 minutes a été réalisée de manière à pouvoir facilement extraire le surnageant limpide.

Après extraction du surnageant, 75 mL d'éthanol + 100 µL d'une solution éthanolique de MEHQ ont été ajoutés dans chacun des pots de centrifugation contenant des culots solides. Ces culots solides ont ensuite été redispersés en solution par l'emploi d'ultrasons, d'agitation magnétique, et d'agitation manuelle.

Une seconde centrifugation à 2594 g pendant 10 minutes a été réalisée de manière à pouvoir facilement extraire le surnageant limpide et ainsi à obtenir un culot solide pouvant ultérieurement être redispersé. Après extraction du surnageant, 75 mL d'éthanol + 100 µL d'une solution éthanoïque de MEHQ ont été ajoutés dans chacun des pots de centrifugation contenant des culots solides. Ces culots solides ont ensuite été redispersés en solution par l'emploi d'ultrasons, d'agitation magnétique, et d'agitation manuelle.

La solution colloïdale a ensuite été reconcentrée jusqu'à atteindre l'extrait sec souhaité à l'aide d'un évaporateur rotatif (40°C, 115-100 mbar, 80-100 rpm). Une suspension colloïdale très stable, de fluorure d'ytterbium fonctionnalisé avec du phosphonate-diméthacrylate, a été obtenue à un extrait sec supérieure à 30% massique en éthanol.

## Revendications

1. Suspension colloïdale de nanoparticules de fluorure de terre rare dans laquelle les nanoparticules de fluorure de terre rare ont une taille donnée en D50 inférieure à 300 nm, de préférence inférieure à 200 nm, et encore plus préférentiellement inférieure à 100 nm, et sont présentes à une concentration supérieure à 20%, le pourcentage étant basé sur le poids de la suspension.

2. Suspension colloïdale de nanoparticules de fluorure de terre rare selon la revendication 1, dans laquelle les nanoparticules de fluorure de terre rare sont fonctionnalisées.

3. Suspension colloïdale de nanoparticules de fluorure de terre rare selon la revendication 1 ou 2, dans laquelle le solvant dispersant les nanoparticules de fluorure de terre rare est choisi parmi les hydrocarbures, les hydrocarbures halogénés, l'eau, les alcools, les éthers, les glycols, les éthers de glycol, les aldéhydes, les cétones, les acides carboxyliques, les esters, les esters de carbonate, les amines, les amides, les thiols, les solvants organosulfurés, les monomères acryliques et méthacryliques, les monomères vinyliques, les monomères époxydes, les huiles silicone, les organosilanes, les polycarbosilanes et les alkoxysilanes.

4. Suspension colloïdale de nanoparticules de fluorure de terre rare selon la revendication 3 dans laquelle le solvant dispersant est choisi parmi l'acétone, l'eau, l'éthanol, le Bisphénol A glycerolate diméthacrylate (Bis-GMA), le bisphénol A glycol éthoxylé diméthacrylate (Bis-EMA), le triéthylène glycol diméthacrylate (TEGDMA), le 2-Hydroxyéthyl méthacrylate (HEMA), le 1,6-Hexanediol diméthacrylate (HDDMA), le 1,10-décanediol diméthacrylate (D3MA) ou un mélange de ces solvants.

5. Procédé de préparation d'une suspension colloïdale de nanoparticules de fluorure de terre rare selon l'une quelconque des revendications 1 à 4 dans lequel :
a. on prépare une solution, de préférence aqueuse, d'un sel de la terre rare correspondante,
b. on prépare une solution d'un complexe de transfert de charge de formule I 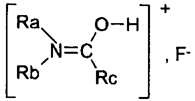 par réaction d'un amide de formule II 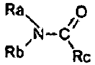 avec une source d'ions fluorure, de préférence de l'acide fluorhydrique, du fluorure de tétrabutylammonium, du trihydrofluorure de triéthylamine ou de l'acide fluorosilicique,
où : Ra et Rb, identiques ou différents, représentent, chacun indépendamment l'un de l'autre, un groupe (C₁-C₆)alkyle, (C₃-C₇)cycloalkyle, phényle ou hétérocycloalkyle de 5 ou 6 chainons, ledit groupe pouvant éventuellement être substitué, ou bien Ra et Rb sont liés entre eux et forment une chaine alkylène, comprenant de 2 à 6 atomes de carbone, éventuellement substituée, et Rc représente un atome d'hydrogène ou un groupe (C₁-C₆)alkyle, (C₃-C₇)cycloalkyle, phényle ou hétérocycloalkyle de 5 ou 6 chaînons, ledit groupe pouvant éventuellement être substitué, ou bien Rb et Rc sont liés entre eux et forment une chaine alkylène, comprenant de 2 à 5 atomes de carbone, éventuellement substituée,
c. on fait réagir dans un solvant ou mélange de solvants, la solution d'un sel de la terre rare correspondante obtenue à l'étape a) et la solution d'un complexe de transfert de charge de formule (I) obtenue à l'étape b) 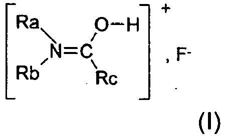
d. on chauffe dans un récipient hermétiquement fermé le mélange obtenu à l'étape c) à une température comprise entre 70°C et 190°C, de manière à obtenir une suspension colloïdale de nanoparticules de fluorure de terre rare,
e. optionnellement, on lave la suspension colloïdale de nanoparticules de fluorure de terre rare, de préférence par au moins un cycle de centrifugation/redispersion dans un solvant approprié jusqu'à ce que le surnageant soit limpide et incolore, étant entendu que le dernier solvant de redispersion est choisi en fonction du solvant d'utilisation de la suspension colloïdale de nanoparticules de fluorure de terre rare.

6. Procédé de préparation d'une suspension colloïdale de nanoparticules de fluorure de terre rare fonctionnalisées suivant l'une quelconque des revendications 2 à 4 dans lequel :
a. on prépare une suspension colloïdale de nanoparticules de fluorure de terre rare selon la revendication 5,
b. on greffe à la surface des nanoparticules de fluorure de terre rare une molécule présentant une structure du type A-Sp-Z dans laquelle :
i. A est une fonction assurant l'accrochage de la molécule à la surface,
ii. Sp est un groupe espaceur,
iii. Z est une fonction chimique permettant de modifier le caractère de surface des nanoparticules.
c. optionnellement, on lave la suspension colloïdale de nanoparticules de fluorure de terre rare fonctionnalisées, de préférence par au moins un cycle de centrifugation/redispersion dans un solvant approprié jusqu'à ce que le surnageant soit exempt de molécules fonctionnalisantes libres, étant entendu que le dernier solvant de redispersion est choisi en fonction du solvant d'utilisation de la suspension colloïdale de nanoparticules de fluorure de terre rare fonctionnalisées.

7. Procédé selon l'une quelconque des revendications précédentes **caractérisé en ce que** la terre rare est choisie parmi le lanthane, le cérium, le praséodyme, le néodyme, le prométhium, le samarium, l'europium, le gadolinium, le terbium, le dysprosium, l'holmium, l'erbium, le thulium, l'ytterbium, le lutécium, l'yttrium et le scandium.

8. Utilisation d'une suspension colloïdale de nanoparticules de fluorure de terre rare selon l'une quelconque des revendications 1 à 4, dans la fabrication d'un matériau de restauration dentaire, et notamment pour le comblement dentaire.

9. Utilisation d'une composition comprenant une suspension colloïdale de nanoparticules de fluorure de terre rare selon l'une quelconque des revendications 1 à 4, dans la fabrication d'un matériau de restauration dentaire, et notamment pour le comblement dentaire.

10. Composition comprenant au moins une suspension colloïdale de nanoparticules de fluorure de terre rare selon l'une quelconque des revendications 1 à 4.

11. Matériau de restauration dentaire polymérisable comprenant :
- une suspension colloïdale de nanoparticules de fluorure de terre rare selon l'une quelconque des revendications 1 à 4,
- au moins un monomère polymérisable,
- un système d'initiateur de polymérisation radicalaire pour le durcissement thermique ou le photodurcissement dudit composite dentaire polymérisable,
- et des charges.

12. Composite dentaire polymérisable comprenant :
(i) de 9 à 29% en poids de résine organique comprenant au moins un monomère polymérisable et une suspension colloïdale de nanoparticules de fluorure de terre rare selon l'une quelconque des revendications 1 à 4,
(ii) de 70% à 90% en poids de charges.
(iii) 1% en poids de composés additionnels comprenant au moins un système initiateur de polymérisation radicalaire pour le durcissement thermique ou le photodurcissement dudit composite dentaire polymérisable.

13. Composite dentaire polymérisable, selon la revendication 12, comprenant parmi les composés additionnels des inhibiteurs de polymérisation et des pigments.

## Patentansprüche

1. Kolloidale Suspension von Nanopartikeln auf Seltenerdfluoridbasis, wobei die Nanopartikel auf Seltenerdfluoridbasis eine in D50 angegebene Größe unter 300 nm, vorzugsweise unter 200 nm und noch vorzugsweiser unter 100 nm haben und in einer Konzentration von über 20 % vorhanden sind, wobei der Prozentsatz auf dem Gewicht der Suspension basiert.

2. Kolloidale Suspension von Nanopartikeln auf Seltenerdfluoridbasis nach Anspruch 1, wobei die Nanopartikel auf Seltenerdfluoridbasis funktionalisiert sind.

3. Kolloidale Suspension von Nanopartikeln auf Seltenerdfluoridbasis nach Anspruch 1 oder 2, wobei das die Nanopartikel auf Seltenerdfluoridbasis dispergierende Lösungsmittel aus den Kohlenwasserstoffen, den halogenierten Kohlenwasserstoffen, Wasser, den Alkoholen, den Ethern, den Glycolen, den Glycolethern, den Aldehyden, den Ketonen, den Carbonsäuren, den Estern, den Carbonatestern, den Aminen, den Amiden, den Thiolen, den Organoschwefellösungsmitteln, den Acryl- und Methacrylmonomeren, den Vinylmonomeren, den Epoxidmonomeren, den Silikonölen, den Organosilanen, den Polycarbosilanen und den Alkoxysilanen ausgewählt ist.

4. Kolloidale Suspension von Nanopartikeln auf Seltenerdfluoridbasis nach Anspruch 3, wobei das dispergierende Lösungsmittel aus Aceton, Wasser, Ethanol, Bisphenol A-Glycidylmethacrylat (Bis-GMA), Bisphenol A-Diethylmethacrylat (Bis-EMA), Triethylenglycoldimethacrylat (TEGDMA), 2-Hydroxyethylmethacrylat (HEMA), 1,6-Hexanedioldimethacrylat (HDDMA), 1,10-Decanedioldimethacrylat (D3MA) oder einem Gemisch dieser Lösungsmittel ausgewählt ist.

5. Verfahren zur Herstellung einer kolloidalen Suspension von Nanopartikeln auf Seltenerdfluoridbasis nach einem der Ansprüche 1 bis 4, wobei:
a. eine vorzugsweise wässrige Lösung eines Salzes der entsprechenden Seltenen Erde hergestellt wird,
b. eine Lösung eines Charge-Transfer-Komplexes der Formel I 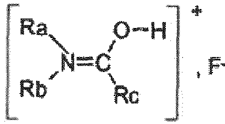 durch Reaktion eines Amids der Formel II 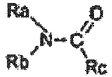 mit einer Fluoridionenquelle, vorzugsweise Fluorwasserstoffsäure, Tetrabutylammoniumfluorid, Triethylamintrihydrofluorid oder Fluorkieselsäure hergestellt wird,
wobei: Ra und Rb, identisch oder unterschiedlich, jeweils unabhängig voneinander eine (C₁-C₆)Alkyl-, (C₃-C₇)Cycloalkyl-, Phenyl- oder Heterocycloalkylgruppe mit 5 oder 6 Kettengliedern darstellen, wobei die Gruppe eventuell substituiert sein kann, oder Ra und Rb miteinander verbunden sind und eine eventuell substituierte Alkylenkette bilden, umfassend 2 bis 6 Kohlenstoffatome, und Rc ein Wasserstoffatom oder eine (C₁-C₆)Alkyl-, (C₃-C₇)Cycloalkyl-, Phenyl- oder Heterocycloalkylgruppe mit 5 oder 6 Kettengliedern darstellt, wobei die Gruppe eventuell substituiert sein kann, oder Rb und Rc miteinander verbunden sind und eine eventuell substituiert Alkylenkette bilden, umfassend 2 bis 5 Kohlenstoffatome,
c. die Lösung eines Salzes der entsprechenden Seltenen Erde aus Schritt a) und die Lösung eines Charge-Transfer-Komplexes der Formel (I) aus Schritt b) 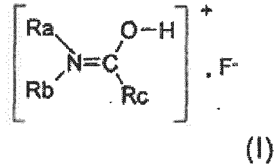 in einem Lösungsmittel oder einem Lösungsmittelgemisch in Reaktion versetzt werden,
d. das erhaltene Gemisch aus Schritt c) bei einer Temperatur zwischen 70 °C und 190 °C derart in einem hermetisch verschlossenen Behältnis erhitzt wird, dass eine kolloidale Suspension von Nanopartikeln auf Seltenerdfluoridbasis erhalten wird,
e. die kolloidale Suspension von Nanopartikeln auf Seltenerdfluoridbasis optional vorzugsweise anhand mindestens eines Zentifugierungs-/Redispersionszyklus in einem geeigneten Lösungsmittel gewaschen wird, bis der Überstand klar und farblos ist, wobei es sich versteht, dass das letzte Redispersions-Lösungsmittel in Abhängigkeit vom Verwendungs-Lösungsmittel der kolloidalen Suspension von Nanopartikeln auf Seltenerdfluoridbasis ausgewählt ist.

6. Verfahren zur Herstellung einer kolloidalen Suspension funktionalisierter Nanopartikel auf Seltenerdfluoridbasis nach einem der Ansprüche 2 bis 4, wobei:
a. eine kolloidale Suspension von Nanopartikeln auf Seltenerdfluoridbasis nach Anspruch 5 hergestellt wird,
b. auf die Oberfläche der Nanopartikel auf Seltenerdfluoridbasis ein Molekül gepfropft wird, das eine Struktur vom Typ A-Sp-Z aufweist, wobei:
i. A eine Funktion ist, die das Haften des Moleküls auf der Oberfläche sichert,
ii. Sp eine Spacergruppe ist,
iii. Z eine chemische Funktion ist, die erlaubt, den Oberflächencharakter der Nanopartikel zu verändern,
c. die kolloidale Suspension funktionalisierter Nanopartikel auf Seltenerdfluoridbasis optional vorzugsweise anhand mindestens eines Zentifugierungs-/Redispersionszyklus in einem geeigneten Lösungsmittel gewaschen wird, bis der Überstand frei von freien funktionalisierenden Molekülen ist, wobei es sich versteht, dass das letzte Redispersions-Lösungsmittel in Abhängigkeit vom Verwendungs-Lösungsmittel der kolloidalen Suspension funktionalisierter Nanopartikel auf Seltenerdfluoridbasis ausgewählt ist.

7. Verfahren nach einem der vorangehenden Ansprüche, **dadurch gekennzeichnet, dass** die Seltene Erde aus Lanthan, Cerium, Praseodym, Neodym, Promethium, Samarium, Europium, Gadolinium, Terbium, Dysprosium, Holmium, Erbium, Thulium, Ytterbium, Lutecium, Yttrium und Scandium ausgewählt ist.

8. Verwendung einer kolloidalen Suspension von Nanopartikeln auf Seltenerdfluoridbasis nach einem der Ansprüche 1 bis 4 zur Herstellung eines Materials zur Zahnwiederherstellung und insbesondere für die Zahnfüllung.

9. Verwendung einer Zusammensetzung, die eine kolloidale Suspension von Nanopartikeln auf Seltenerdfluoridbasis nach einem der Ansprüche 1 bis 4 umfasst, zur Herstellung eines Materials zur Zahnwiederherstellung und insbesondere für die Zahnfüllung.

10. Zusammensetzung, umfassend mindestens eine kolloidale Suspension von Nanopartikeln auf Seltenerdfluoridbasis nach einem der Ansprüche 1 bis 4.

11. Polymerisierbares Material zur Zahnwiederherstellung, umfassend:
- eine kolloidale Suspension von Nanopartikeln auf Seltenerdfluoridbasis nach einem der Ansprüche 1 bis 4,
- mindestens ein polymerisierbares Monomer,
- ein Initiatorsystem einer Radikalpolymerisation für die thermische Härtung oder die Photohärtung des polymerisierbaren Zahnkomposits,
- und Chargen.

12. Polymerisierbares Zahnkomposit, umfassend:
(i) 9 bis 29 Gew.-% organisches Harz, umfassend mindestens ein poylmerisierbares Monomer und eine kolloidale Suspension von Nanopartikeln auf Seltenerdfluoridbasis nach einem der Ansprüche 1 bis 4,
(ii) 70 bis 90 Gew.-% Chargen,
(iii) 1 Gew.-% zusätzliche Verbindungen, die mindestens ein Initiatorsystem einer Radialpolymerisation für die thermische Härtung oder die Photohärtung des polymerisierbaren Zahnkomposits umfassen.

13. Polymerisierbares Zahnkomposit nach Anspruch 12, umfassend unter den zusätzlichen Verbindungen Polymerisationshemmer und Pigmente.

## Claims

1. Colloidal suspension of rare earth fluoride nanoparticles wherein the rare earth fluoride nanoparticles have a size given in D50 of less than 300 nm, preferably less than 200 nm, and even more preferably less than 100 nm, and are present at a concentration of more than 20%, the percentage being based on the weight of the suspension.

2. Colloidal suspension of rare earth fluoride nanoparticles according to claim 1, wherein the rare earth fluoride nanoparticles are functionalised.

3. Colloidal suspension of rare earth fluoride nanoparticles according to claim 1 or 2, wherein the solvent dispersing the rare earth fluoride nanoparticles is chosen from hydrocarbons, halogenated hydrocarbons, water, alcohols, ethers, glycols, glycol ethers, aldehydes, ketones, carboxylic acids, esters, carbonate esters, amines, amides, thiols, organosulphur solvents, acrylic and methacrylic monomers, vinyl monomers, epoxide monomers, silicone oils, organosilanes, polycarbosilanes and alkoxysilanes.

4. Colloidal suspension of rare earth fluoride nanoparticles according to claim 3, wherein the dispersing solvent is chosen from acetone, water, ethanol, Bisphenol A glycerolate dimethacrylate (Bis-GMA), Bisphenol A glycol ethoxylate dimethacrylate (Bis-EMA), triethylene glycol dimethacrylate (TEGDMA), 2-Hydroxyethyl methacrylate (HEMA), 1,6-Hexanediol dimethacrylate (HDDMA), 1,10-decanediol dimethacrylate (D3MA) or a mixture of these solvents.

5. Method for preparing a colloidal suspension of rare earth fluoride nanoparticles according to any one of claims 1 to 4 wherein:
a. a solution, preferably aqueous, of a corresponding rare earth salt is prepared,
b. a solution of a charge transfer complex of formula I 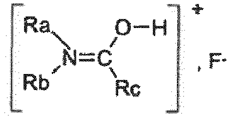 is prepared by reacting an amide of formula II 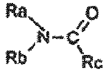 with a source of fluoride ions, preferably hydrofluoric acid, tetrabutylammonium fluoride, triethylamine trihydrofluoride or fluorosilicic acid,
where: Ra and Rb, identical or different, represent, each independently of one another, a (C₁-C₆)alkyl, (C₃-C₇)cycloalkyl, phenyl or heterocycloalkyl group of 5 or 6 links, said group optionally being substituted, or Ra and Rb are bonded to one another and form an alkylene chain, comprising from 2 to 6 carbon atoms, optionally substituted, and Rc represents a hydrogen atom or a (C₁-C₆)alkyl, (C₃-C₇)cycloalkyl, phenyl or heterocycloalkyl group of 5 or 6 links, said group optionally being substituted, or Rb and Rc are bonded to one another and form an alkylene chain, comprising from 2 to 5 carbon atoms, optionally substituted,
c. in a solvent or solvent mixture, the solution of a corresponding rare earth salt obtained in step a) is reacted with the solution of a charge transfer complex of formula (I) obtained in step b) 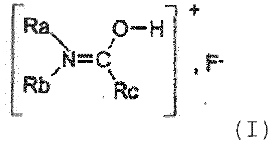
d. in a hermetically sealed receptacle, the mixture obtained in step c) is heated to a temperature between 70°C and 190°C, so as to obtain a colloidal suspension of rare earth fluoride nanoparticles,
e. optionally, the colloidal suspension of rare earth fluoride nanoparticles is washed, preferably with at least one centrifugation/redispersion cycle in a suitable solvent until the supernatant is clear and colourless, it being understood that the last redispersion solvent is chosen according to the solvent of use of the colloidal suspension of rare earth fluoride nanoparticles.

6. Method for preparing a colloidal suspension of functionalised rare earth fluoride nanoparticles according to any one of claims 2 to 4 wherein:
a. a colloidal suspension of rare earth fluoride nanoparticles is prepared according to claim 5,
b. a molecule having a structure of the type A-Sp-Z is grafted on the surface of the rare earth fluoride nanoparticles, wherein:
i. A is a function bonding the molecule to the surface,
ii. Sp is a spacer group,
iii. Z is a chemical function for modifying the surface property of the nanoparticles.
c. optionally, the colloidal suspension of functionalised rare earth fluoride nanoparticles is washed, preferably with at least one centrifugation/redispersion cycle in a suitable solvent until the supernatant is free from free functionalising molecules, it being understood that the last redispersion solvent is chosen according to the solvent of use of the colloidal suspension of functionalised rare earth fluoride nanoparticles.

7. Method according to any one of the preceding claims **characterised in that** the rare earth is chosen from lanthanum, cerium, praseodymium, neodymium, promethium, samarium, europium, gadolinium, terbium, dysprosium, holmium, erbium, thulium, ytterbium, lutecium, yttrium and scandium.

8. Use of a colloidal suspension of rare earth fluoride nanoparticles according to any one of claims 1 to 4, in the manufacture of a dental restoration material, and particularly for dental filling.

9. Use of a composition comprising a colloidal suspension of rare earth fluoride nanoparticles according to any one of claims 1 to 4, in the manufacture of a dental restoration material, and particularly for dental filling.

10. Composition comprising at least one colloidal suspension of rare earth fluoride nanoparticles according to any one of claims 1 to 4.

11. Polymerisable dental restoration material comprising:
- a colloidal suspension of rare earth fluoride nanoparticles according to any one of claims 1 to 4,
- at least one polymerisable monomer,
- a radical polymerisation initiator system for the thermal hardening or photohardening of said polymerisable dental composite,
- and fillers.

12. Polymerisable dental composite comprising:
(i) from 9 to 29% by weight of organic resin comprising at least one polymerisable monomer and a colloidal suspension of rare earth fluoride nanoparticles according to any one of claims 1 to 4,
(ii) from 70% to 90% by weight of fillers,
(iii) 1% by weight of additional compounds comprising at least one radical polymerisation initiator system for the thermal hardening or photohardening of said polymerisable dental composite.

13. Polymerisable dental composite, according to claim 12, comprising among the additional compounds polymerisation inhibitors and pigments.
